# FASCICULE DE BREVET EUROPEEN

(11) **EP 1 773 284 B1**
(45) Date de publication et mention de la délivrance du brevet: **15.09.2010**
(21) Numéro de dépôt: 05796258.1
(22) Date de dépôt: 05.08.2005
(51) Int. Cl.: A61K 8/00

(54) **TETRAHYDROPYRAN(ON)ES SUBSTITUES EN BETA, LEUR PROCEDE DE SYNTHESE ET LEUR UTILISATION EN PARFUMERIE**
BETA-SUBSTITUIERTE TETRAHYDROPYRAN(ON)E UND DEREN VEHRFAREN ZUR HERSTELLUNG SOWIE DEREN VERWENDUNG IN DER PARFÜMERIE
BETA-SUBSTITUTED TETRAHYDROPYRAN(ON)S AND METHOD FOR THE SYNTHESIS AND THEIR USE IN PERFUMERY

(30) Priorité: 06.08.2004 FR 0408729
(43) Date de publication de la demande: 18.04.2007
(73) Titulaire: V. Mane Fils, 06620 Bar sur Loup (FR)
(72) Inventeur: MANE, Jean, F-06130 Grasse (FR); CHANOT, Jean-Jacques, F-06530 Speracedes (FR); SCHROEDER, Martin, Kent TN23 5GE (GB)
(74) Mandataire: Cabinet Plasseraud
(86) Numéro de dépôt international: PCT/FR2005/002038
(87) Numéro de publication internationale: WO 2006/021663

(56) Documents cités:
- DE-A1- 10 353 656
- DE-A1- 10 353 658
- US-A- 4 288 350
- CORBIN, DALE H. ET AL: "Stereochemical assignments of the 7-phenyl-3-oxabicyclo[4.1.0]heptanes" JOURNAL OF HETEROCYCLIC CHEMISTRY , 18(3), 643-4 CODEN: JHTCAD; ISSN: 0022-152X, 1981, XP001205571
- WOODYARD J D ET AL: "THE SYNTHESIS OF THE BENZYLTETRAHYDROPYRANS" JOURNAL OF HETEROCYCLIC CHEMISTRY, PROVO, UT, US, vol. 13, no. 3, juin 1976 (1976-06), pages 647-648, XP001205569 ISSN: 0022-152X
- GEVORKYAN, A. A. ET AL: "Cycloalkylation of acetylenic and allenic hydrocarbons with bis(halomethyl) ethers. New synthesis of pyran derivatives" DOKL. VSES. KONF. KHIM. ATSETILENA, 4TH , VOLUME 1, 152-7 FROM: REF. ZH., KHIM. 1973, ABSTR. NO. 4ZH288, 1972, XP009045120
- METZGER, JUERGEN O. ET AL: "Aluminum chloride-induced additions of formaldehyde to alkenes" BULLETIN DES SOCIETES CHIMIQUES BELGES , 103(7-8), 393-7 CODEN: BSCBAG; ISSN: 0037-9646, 1994, XP009045126
- VOLYNSKII, N. P. ET AL: "Preparative synthesis of 3-alkyl(cycloalkyl)thiacyclohexanes" IZVESTIYA AKADEMII NAUK SSSR, SERIYA KHIMICHESKAYA , (10), 2299-302 CODEN: IASKA6; ISSN: 0002-3353, 1976, XP009045115
- PENG, NAN ET AL: "Composition of volatile oil of Primula obconica in central China" NATURAL PRODUCT LETTERS , 16(4), 249-253 CODEN: NPLEEF; ISSN: 1057-5634, 2002, XP000945122
- BUTLER, GEORGE B.: "Radical cyclo- and cyclocopolymerization" JOURNAL OF POLYMER SCIENCE, POLYMER SYMPOSIA , 64(UNSOLVED PROBL. CO- GRAFT POLYM.), 71-93 CODEN: JPYCAQ; ISSN: 0360-8905, 1978, XP009045116
- HARTUNG, JENS ET AL: "Towards improved alkoxyl radical precursors. The synthesis of N-alkoxy-4-(4-chlorophenyl)thiazole-2(3H)- thiones" SYNLETT , (7), 848-850 CODEN: SYNLES; ISSN: 0936-5214, 1997, XP001205572
- KAYSER, MARGARET M. ET AL: "Enantioselective and Regioselective Baeyer-Villiger Oxidation of 2- and 3-Substituted Cyclopentanones Using Engineered Bakers' Yeast" JOURNAL OF ORGANIC CHEMISTRY , 63(20), 7103-7106 CODEN: JOCEAH; ISSN: 0022-3263, 1998, XP001205391
- CRICH, DAVID ET AL: "Generation and Trapping of Alkene Radical Cations under Nonoxidizing Conditions: Formation of Six-Membered Rings by Exo- and Endo-Mode Cyclizations" ORGANIC LETTERS , 4(15), 2573-2575 CODEN: ORLEF7; ISSN: 1523-7060, 2002, XP001205566
- LEMOULT, STEPHANIE C. ET AL: "Lipase-catalyzed Baeyer-Villiger reactions" JOURNAL OF THE CHEMICAL SOCIETY, PERKIN TRANSACTIONS 1: ORGANIC AND BIO-ORGANIC CHEMISTRY , (2), 89-91 CODEN: JCPRB4; ISSN: 0300-922X, 1995, XP002928285
- YAMAGUCHI, MASAHIKO ET AL: "A new synthesis of .delta.-lactones from oxetanes" TETRAHEDRON LETTERS , 25(11), 1159-62 CODEN: TELEAY; ISSN: 0040-4039, 1984, XP001205395
- WANG S ET AL: "ACCESS TO OPTICALLY PURE 4- AND 5-SUBSTITUTED LACTONES: A CASE OF CHEMICAL-BIOCATALYTICAL COOPERATION" JOURNAL OF ORGANIC CHEMISTRY, AMERICAN CHEMICAL SOCIETY. EASTON, US, vol. 68, no. 16, 15 juillet 2003 (2003-07-15), pages 6222-6228, XP001205390 ISSN: 0022-3263

## Description

La présente invention concerne d'une manière générale le domaine des agents odorants, et leur utilisation notamment en parfumerie, en particulier dans les compositions topiques et les produits d'entretien. En particulier, la présente invention concerne l'utilisation comme agents odorants de composés tétrahydropyra(no)nes - c'est-à-dire tétrahydropyrannes et tétrahydropyranones - substitués en position bêta par rapport à l'oxygène du cycle, du fait de leurs notes agrumes, verveine, fruitées ou nitriles, et concerne également leur procédé de synthèse.

Le terme parfumerie est ici utilisé pour désigner non seulement la parfumerie au sens habituel du terme, mais également les autres domaines dans lesquels l'odeur des produits est importante. Il peut s'agir de compositions de parfumerie au sens habituel du terme, telles que bases et concentrés parfumants, eaux de Cologne, eaux de toilette, parfums et produits similaires; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, antitranspirants et désodorisants corporels, lotions et crèmes de rasage, savons, crèmes, dentifrices, bains de bouche, pommades, et produits similaires; et de produits d'entretien, tels qu'assouplissants, détergents, lessives, désodorisants d'ambiance, et produits similaires.

Le terme odorant est utilisé ici pour désigner un composé qui exhale une odeur.

Certaines lactones (c'est-à-dire tétrahydropyranones) et certains pyrannes sont déjà utilisés en parfumerie. La plupart des lactones utilisées en parfumerie sont des dérivés d'acides gras et donc substituées en position 6, comme la γ-décalactone ou la γ-dodécalactone qui apportent une note fruitée, balsamique aux compositions odorantes. Il en est de même pour l'utilisation des pyrannes en parfumerie, qui sont également substitués en position alpha par rapport à l'oxygène incorporé dans le cycle. Pour n'en mentionner que deux : le rose oxyde et le Doremox® de chez Firmenich apportent des notes typiques pour les compositions de rose.

Le brevet américain US 4 288 350 décrit des 5-dialkyl-tetrahydro-2H-pyran-2-ones (γγ-dialkyl-δ-valérolactones)ayant une odeur d'huile de racine de costus.

Il subsiste un besoin pour d'autres agents odorants, afin d'étendre la gamme des notes pouvant être apportées à une composition et les options disponibles pour ajouter ces notes.

Par ailleurs, les procédés de synthèse connus peuvent être améliorés. Les tetrahydropyranones ont souvent été synthétisées via l'oxydation enzymatique de la cétone correspondante comme décrit par Margaret M. Kayser et al, Journal of Organic Chemistry (1998), 63 (20), 7103-7106 *;* et Stephanie C. Lemoult et al, Journal of the Chemical Society, Perkin Transactions 1 : Organic and Bio-Organic Chemistry (1995), (2), 89-91*.* Chimiquement, elles sont synthétisées à partir des oxétanes (Masahiko Yamaguchi et al, Tetrahedron Letters (1984), 25 (11), 1159-62*)* ou via une réaction Mukalyama-Michael entre silylcétènes acétals et alkylidènemalonates *(*David A. Evans et al, J. Am. Chem. Soc. (1999), 121 (9), 1994-1995*).*

Quatre chemins principaux de synthèse ont été employés pour la synthèse des tetrahydropyrannes : La réaction de Prins, comme déjà décrit par Paul R. Straps dans J. Org. Chem ; (1969), 34 (3), 479-85*,* l'addition radicalaire de dérivés acétylénés au tetrahydropyranne, *(*Montaudon, E. et al, Bull. Soc. Chim. Fr. (1974), 11, Pt. 2, 2635-8*)* ou via la cyclisation d'un diol, synthétisé à partir d'un diacide comme décrite par E. Montaudon et al, (J. Heterocycl. Chem. (1979), 16 (11), 113-21) et Paul R. Strapp dans la publication mentionnée ci-dessus.

Cependant, ces procédés sont difficiles à mettre en oeuvre à une échelle industrielle. Il était donc nécessaire de trouver une voie de synthèse susceptible d'être industrialisée tout en donnant accès à un grand nombre de composés différents.

L'invention a pour objet un agent odorant constitué d'un composé tétrahydropyra(no)ne substitué en position bêta par rapport à l'oxygène du cycle, représenté par la formule suivante : 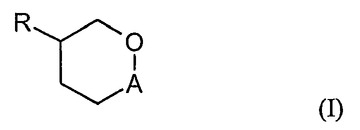 dans laquelle le substituant R représente un radical alkyle linéaire CH₃-(CH₂)ₙ- avec n = 2 à 10 inclus, (CH₃)₂CH- ou C₆H₅-(CH₂)ₘ-, avec m = 0 ou 1; ou 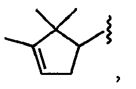 et dans laquelle A représente -CH₂- ou -CO-.

Plus spécifiquement, l'invention a pour objet un agent odorant constitué d'un composé tétrahydro-2H-pyran-2-one ou tétrahydropyranne, substitué en position bêta par rapport à l'oxygène du cycle, de formule (Ia) et (Ib), respectivement : 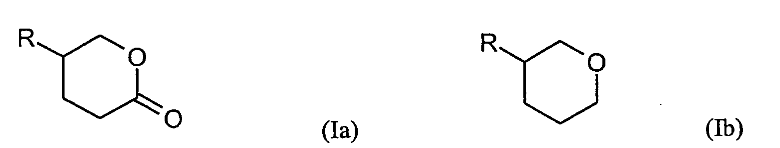 dans lesquelles R a la même signification que dans la formule (I).

La formule (Ia) correspond à la formule (I) dans laquelle A représente -CO-.

La formule (Ib) correspond à la formule (I) dans laquelle A représente -CH2-.

Autrement dit, l'invention a pour objet l'utilisation comme agent odorant d'un composé de formule (I), (Ia) ou (Ib).

Dans certains modes de réalisation, R représente dans la formule (I), (Ia) ou (Ib), un radical alkyle linéaire CH₃-(CH₂)ₙ- avec n = 4 à 10 inclus. Dans certains modes de réalisation, R représente dans cette formule un radical alkyle linéaire CH₃-(CH₂)ₙ- avec n = 4 à 8 inclus, ou encore un radical alkyle linéaire CH₃-(CH₂)ₙ- avec n = 6 à 10 inclus.

En particulier, l'invention a pour objet un agent odorant choisi parmi les agents odorants suivants, autrement dit l'utilisation comme agent odorant d'une des molécules suivantes :
5-pentyl-tétrahydro-pyran-2-one,
3-heptyl-tétrahydro-pyranne,
5-heptyl-tétrahydro-pyran-2-one,
3-benzyl-tetrahydro-pyranne,
les substituants correspondant à R dans la formule (I) étant linéaires.

Parmi les agents odorants représentés en formule (I), certains sont, à la connaissance des inventeurs, des molécules nouvelles qui sont donc revendiquées en tant que telles. Il s'agit de :
5-pentyl-tétrahydro-pyran-2-one,
5-nonyl-tétrahydro-pyran-2-one,
5-benzyl-tétrahydro-pyran-2-one,
5-(2,2,3-triméthyl-cyclopent-3-enyl)-tétrahydro-pyran-2-one,
3-heptyl-tétrahydro-pyranne,
3-octyl-tétrahydro-pyranne,
3-nonyl-tétrahydro-pyranne,
3-decyl-tétrahydro-pyranne,
3-undecyl-tétrahydro-pyranne, et
3-(2,2,3-triméthyl-cyclopent-3-enyl)-tétrahydro-pyranne,
les substituants correspondant à R dans la formule (I) étant linéaires.

Autrement dit, l'invention a pour objet les molécules de formule (I) 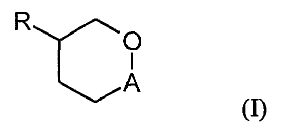 dans laquelle
le substituant R représente un radical alkyle linéaire CH₃-(CH₂)ₙ- avec n = 6 à 10 inclus,
ou 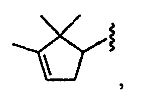 lorsque A représente -CH₂- ,
et
le substituant R représente un radical alkyle linéaire CH₃-(CH₂)ₙ- avec n = 4, 6 ou 8, ou C₆H₅-
ou 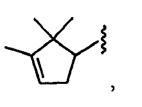 lorsque A représente -CO-.

A la connaissance des inventeurs, aucun des composés représentés en formule I n'a à ce jour été décrit comme ayant des propriétés odorantes, et aucun n'a à ce jour été utilisé en parfumerie.

L'invention a également pour objet une composition comprenant un produit de base et une quantité efficace d'un composé de formule (I) tel que défini ci-dessus.

Il peut s'agir d'une composition parfumante dans laquelle l'agent odorant est utilisé pour masquer ou neutraliser certaines odeurs, ou encore pour améliorer significativement la note olfactive de cette même composition. En effet, l'agent odorant de formule (I) apporte à la composition parfumante, qui la comporte une note olfactive plus riche, plus dynamique, plus intense, plus large, que la même composition parfumante sans sa présence. Ladite composition peut être choisie parmi les compositions de parfumerie au sens habituel du terme, telles que bases et concentrés parfumants, eaux de Cologne, eaux de toilette, parfums et produits similaires; les compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, antitranspirants et désodorisants corporels, lotions et crèmes de rasage, savons, crèmes, dentifrices, bains de bouche, pommades, et produits similaires; et les produits d'entretien, tels qu'assouplissants, détergents, lessives, désodorisants d'ambiance, et produits similaires.

Le produit de base sera aisément déterminé par l'homme du métier en fonction de la composition envisagée et donc de l'utilisation envisagée, pour lesquelles les composants habituels - tels que solvant(s) et/ou adjuvant(s) à titre d'exemple non limitatifs - sont bien connus.

La quantité efficace du composé de formule (I) incorporé dans la composition variera selon le composé, la nature de la composition, l'effet odorant souhaité, et la nature des autres composés odorants ou non éventuellement présents, et pourra être aisément déterminée par l'homme du métier, sachant qu'elle peut varier dans une plage très étendue, de 0,1 à 99% en poids, en particulier 0,1 à 50% en poids, notamment 0,1 à 30% en poids.

Un composé de formule (I) peut être présent sous forme d'un isomère ou d'un mélange d'isomères, en particulier d'un énantiomère ou d'un mélange d'énantiomères, ou d'un mélange racémique.

Ledit composé-peut-être utilisé comme unique agent odorant ou, comme il est courant en parfumerie, il peut être en mélange avec un ou plusieurs autres composés odorants, que l'homme du métier est à même de choisir en fonction de l'effet recherché. Le ou les agents odorants supplémentaires peuvent être des composés de formule (I) ou d'autres agents odorants connus de l'homme du métier.

Ledit composé peut être utilisé tel quel ou il peut être incorporé dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

L'invention a également pour objet un procédé de synthèse des composés de formule (I). Un schéma de synthèse relatif à un mode de réalisation est présenté ci-dessous. 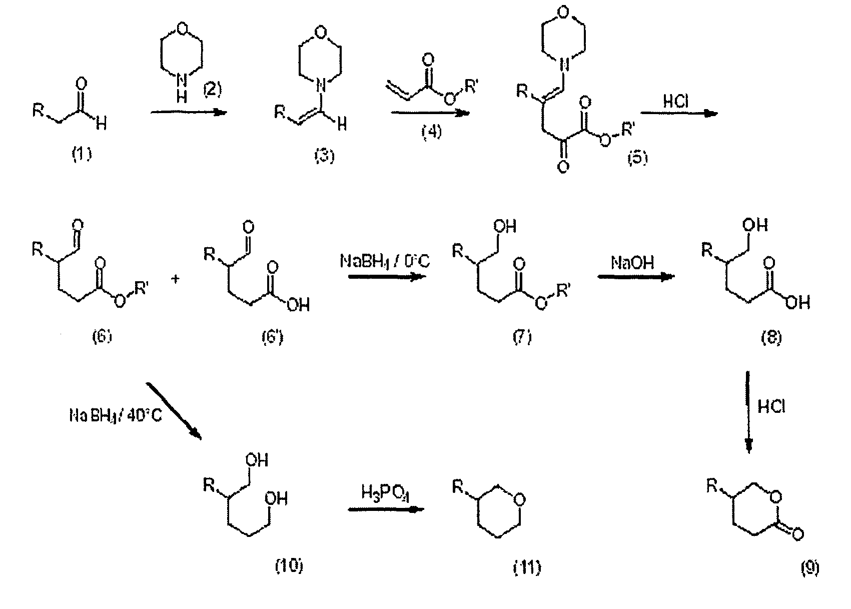 dans lequel R a la même signification que dans la formule (I), (Ia) ou (Ib), et R' représente CH₃ ou C₂H₅ ou un ester d'alcool supérieur, de préférence un produit disponible dans le commerce tel que CH₃ ou C₂H₅ afin de ne pas augmenter le coût de la synthèse.

Sur ce schéma, le composé (9) correspond à un composé de formule (Ia), alors que le composé (11) correspond à un composé de formule (Ib).

Tous les composés de formule (I) peuvent être synthétisés par réduction à partir d'un composé unique oxo-ester de formule (6) 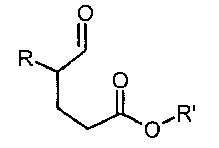 dans laquelle R a la même signification que dans la formule (I) et R' représente CH₃ ou C₂H₅- ou un ester d'alcool supérieur.

L'invention a donc pour objet un procédé de synthèse d'un composé de formule (I) comprenant la réduction d'un composé oxo-ester de formule (6).

En fonction des conditions de la réaction de réduction, on obtient un composé de formule (Ia) ou (Ib).

Le composé oxo-ester (6) peut être synthétisé par un procédé comprenant la condensation d'une amine secondaire avec un aldéhyde, pour former une énamine, suivie de la condensation de l'énamine obtenue avec un acrylate, pour former un énamine ester qui est hydrolysé en oxo-ester. Cette voie de synthèse présente l'avantage de limiter les réactions d'autocondensation de l'aldéhyde.

Dans un mode de réalisation préféré de l'invention, le composé oxo-ester (6) est synthétisé de la façon suivante.

Dans un premier temps, une énamine (3) est formée à partir d'un aldéhyde (1) et d'une amine secondaire. Cette réaction est réalisée de préférence avec une amine cyclique comme la pipéridine ou la morpholine (2), mais d'autres amines, telles que des amines acycliques, peuvent également être employées. La morpholine est intéressante pour des raisons économiques (faible coût). Cette réaction ne nécessite pas l'emploi d'un catalyseur, mais des catalyseurs habituellement utilisés pour la formation d'énamines peuvent être employés. Leur utilisation n'augmente ni le rendement ni le taux de transformation, par contre ils peuvent augmenter la vitesse de la réaction. L'amine secondaire (2) est chauffée dans un solvant comme par exemple le cyclohexane jusqu'à la température à laquelle l'azéotrope du solvant et de l'eau commencerait à distiller. L'aldéhyde (1) est ajouté à cette température, de préférence lentement pour minimiser son auto-condensation. A la fin de la réaction, le solvant et l'excès d'amine secondaire (2) sont évaporés sous pression réduite. L'énamine (3) (qui est une alcényl-morpholine lorsque l'amine secondaire est une morpholine) est directement engagée dans la réaction suivante. Une purification par distillation n'est pas nécessaire et n'augmente pas le rendement de la réaction de Michael qui s'enchaîne.

Une base appropriée telle que l'hydroxyde de potassium dans le méthanol (environ 1 % molaire) est ajoutée à l'énamine (3), et le mélange est de préférence porté à environ 50°C. Un acrylate (4) est ajouté lentement et le milieu réactif est encore agité, de préférence à une température élevée, après la fin de l'addition. On forme ainsi un énamine ester (5). Si la réaction est faite à une température plus basse, la transformation est lente, alors qu'une température plus élevée (de l'ordre de 50°C) favorise la polymérisation de l'acrylate (4) et est donc préférée.

L'énamine ester (5) est ensuite hydrolysé par un acide approprié, pour donner l'oxo-ester (6). L'hydrolyse est faite à une température d'environ 20°C à 80°C, de préférence environ 60°C. L'acide approprié utilisé dans l'hydrolyse de l'énamine ester (5) peut être un acide minéral comme l'acide chlorhydrique ou l'acide sulfurique, ou encore une solution d'acide acétique tamponnée par l'acétate de sodium. Le traitement à l'acide acétique a l'avantage d'être plus doux et permet d'augmenter le rendement de l'oxo-ester (6) par rapport à un traitement avec l'acide chlorhydrique par exemple. Dans les conditions décrites ci-dessus, la saponification partielle de l'oxo-ester (6) en oxo-acide correspondant (6') ne peut pas être complètement évitée. La durée de l'hydrolyse doit donc être soigneusement choisie pour limiter la formation de l'oxo-acide correspondant (6'). En particulier, cette durée est de 2 h à 8 h, de préférence 3 h à 6 h. Le rendement de la réaction est de l'ordrede 60-70%.

L'invention a pour objet un procédé de synthèse d'un composé de formule (I), le procédé comprenant la réduction d'un oxo-ester de formule (6) 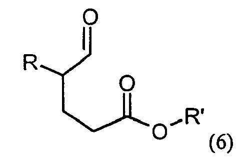 dans laquelle R a la même signification que dans la formule (I) et R' représente CH₃ ou C₂H₅- ou un ester d'alcool supérieur.

Selon les conditions de réduction de l'oxo-ester (6), on obtient soit un hydroxy-ester (7), précurseur d'un dérivé lactonique (9) correspondant à un composé de formule (la), soit un diol (10) qui mène par traitement acide à un pyranne (11) correspondant à un composé de formule (Ib).

L'hydroxyester (7) n'est pas isolé, il est converti en lactone avec un rendement de 60-70 %, généralement 67%.

Les diols sont isolés et convertis en tétrahydropyrannes correspondants avec des rendements de l'ordre de 60-70%. ,

Le rendement global de la synthèse selon l'invention est de l'ordre de 35-45%, tant pour l'obtention des les produits de formule (Ia) que pour les produits de formule (Ib).

L'invention a pour objet un procédé de synthèse d'un composé de formule (I), le procédé comprenant la réduction d'un oxo-ester de formule (6) avec un agent réducteur approprié,
a) à basse température, pour former un hydroxy-ester (7), suivie d'une saponification avec une base appropriée, puis une hydrolyse avec un acide approprié, suivie d'une cyclisation pour former un composé de formule (Ia), ou
b) à haute température, pour former un diol (10), suivie d'une cyclisation pour former un composé de formule (Ib).

Par basse température, on entend une température d'environ -10°C à 10°C, de préférence d'environ -5°C à 5°C, de préférence encore environ 0°C.

Par haute température, on entend une température d'environ 30°C à 50°C, de préférence d'environ 30°C à 40°C, de préférence encore environ 35°C.

Bien entendu, l'homme du métier saura apprécier que la quantité d'agent réducteur nécessaire est variable selon que l'on réduise uniquement l'aldéhyde ou les deux fonctions, et il saura déterminer la quantité appropriée en fonction de l'objectif poursuivi. A titre indicatif, pour la réduction de l'aldéhyde, il est approprié d'ajouter environ 0,25 équivalent (mol), et environ 0,75 équivalent (mol) pour la réduction des deux fonctions.

Un agent réducteur approprié pour la réduction sélective de la fonction aldéhyde peut être le borohydrure de sodium ou les polyméthylhydrosiloxanes ou des boranes. Des réducteurs forts sont nécessaires pour la formation du diol, à titre d'exemples le borohydrure de sodium, l'hydrure de lithium et d'aluminium, le triisopropyloxyde d'aluminium ou simplement du sodium.

Dans un mode de réalisation du procédé selon l'invention, la réduction de l'oxo-ester (6) à l'aide d'un agent réducteur, par exemple le borohydrure de sodium ou polyméthylhydrosiloxane, peut être faite à basse température, c'est-à-dire d'environ -10°C à 10°C, de préférence d'environ -5°C à 5°C, de préférence encore environ 0°C, dans un solvant approprié comme l'éthanol ou le méthanol, avec une quantité d'agent réducteur correspondant à environ 0,2 à 0,3 équivalent molaire, de préférence un peu plus d'environ 0,25 équivalent molaire (éventuellement augmenté de la quantité nécessaire pour neutraliser l'oxo-acide (6') présent dans le milieu réactionnel si l'oxo-ester a été synthétisé par le procédé décrit ci-dessus). On obtient ainsi un hydroxy-ester (7). L'hydroxy-ester peut être saponifié directement, sans effort de récupération, avec une base appropriée telle que l'hydroxyde de sodium en milieu aqueux. La phase aqueuse est alors extraite avec un solvant approprié tel que le toluène pour enlever l'alcool qui se forme à partir de l'aldéhyde d'auto-condensation de l'aldéhyde de départ. Le milieu réactionnel est ensuite acidifié avec un acide approprié tel que l'acide chlorhydrique, ce qui permet d'hydrolyser en même temps les sels de bore et les sels de sodium de l'hydroxy-acide (8). L'hydroxy-acide (8) ne peut pas être isolé car il cyclise en milieu acide spontanément en formant une δ-lactone (9) correspondant à un composé de formule (Ia). La lactone est récupérée et purifiée par distillation.

Dans un autre mode de réalisation du procédé selon l'invention, la réduction de l'oxo-ester grâce à un agent réducteur, par exemple le borohydrure de sodium ou l'hydrure de lithium et d'aluminium, est faite à une température d'environ 30°C à 50°C, de préférence d'environ 30°C à 40°C, de préférence encore environ 35°C, dans un solvant approprié tel que l'éthanol ou l'isopropanol, avec une quantité d'agent réducteur correspondant à environ 0,6 à 1,2 équivalents molaires, de préférence à un peu plus d'un équivalent molaire (éventuellement additionnés de la quantité nécessaire pour neutraliser l'oxo-acide (6') présent dans le milieu réactionnel si l'oxo-ester a été synthétisé par le procédé décrit ci-dessus). La fonction ester est réduite, et on obtient un diol (10). Le milieu réactionnel est ensuite acidifié avec un acide approprié tel que l'acide chlorhydrique, et le milieu aqueux est extrait avec un solvant approprié tel que le toluène pour récupérer le diol. Un diol à faible masse moléculaire, notamment, est bien soluble dans l'eau. On doit donc veiller à ce que la récupération du diol (10) soit quantitative. Le diol (10) est ensuite purifié par distillation, puis on effectue une cyclisation, par exemple en traitant le diol par un acide approprié tel que l'acide phosphorique à 85 %, et à une température appropriée, en particulier entre 120°C et 140°C, pour former un pyranne (11) correspondant à un composé de formule (Ib). Le pyranne (11) est récupéré et purifié par distillation.

Les procédés de synthèse d'un composé de formule (I) comprenant la réduction d'un oxo-ester de formule (6) peuvent inclure les étapes de synthèse de l'oxo-ester de formule (6) décrites ci-dessus en liaison avec les procédés de synthèse dudit-oxo-ester.

Les exemples suivants illustrent davantage les agents odorants selon l'invention, ainsi que leur utilisation, leur synthèse et leur intérêt. Ces exemples ne sont présentés que dans un but d'illustration et ne peuvent être considérés comme limitatifs de la portée de l'invention.

### Exemple 1 : Synthèse du 4-formyl-nonanique ester de méthyle (6):

Dans un ballon de quatre litres avec agitation magnétique, séparateur d'eau, thermomètre et ampoule d'introduction, on charge 418.0 g (4.80 mol) de morpholine et 344.0 g de cyclohexane. Le mélange est porté à une température de 65°C à 70°C. On ajoute en cinq heures 444.2 g (3.89 mol) d'heptanal. On obtient ainsi rapidement la température d'ébullition (80°C-84°C au début d'addition puis 87°C vers la fin de l'opération). L'eau formée pendant la réaction est récupérée dans un séparateur d'eau. Le milieu réactionnel est ensuite refroidi et le cyclohexane et l'excès de la morpholine sont distillés sous pression réduite (environ 4400 Pa) sans dépasser 80°C dans la masse. On refroidit à 40°C, on porte à pression ambiante et on ajoute 4.0 g d'hydroxyde de potassium dans 40 ml de méthanol à la 4-hept-1 (E/Z)-enyl-morpholine. Le mélange est porté à 60°C et 237.5 g (2.76 mol) d'acrylate de méthyl sont ajoutés pendant quatre heures. On agite douze heures à 60°C puis on ajoute encore 165.3 g (1.92 mol) d'acrylate de méthyle en deux heures et trente minutes. Après la fin de l'addition, la température du milieu réactionnel est augmentée à 80°C et l'agitation est poursuivie pendant douze heures. On ajoute goutte à goutte en deux heures à cette température une solution aqueuse d'acide acétique tamponnée à pH 4. (587.3 g acide acétique, 215.3 g acétate de sodium trihydrate et 697.5 g eau). On continue d'agiter pendant encore quatre heures. On laisse décanter et on sépare les phases. La phase aqueuse est extraite deux fois avec 200 ml de cyclohexane. On réunit les phases organiques et on les lave une fois avec 100 ml d'eau, une fois avec 100 ml d'une solution saturée de bicarbonate de sodium et une fois avec 150 ml d'eau salée. On sèche sur sulfate de magnésium. Après évaporation du solvant sous pression réduite (3990 Pa) sans dépasser 50°C dans la masse, on obtient 890.0 g d'oxo-ester brut qui est blanchi sous vide poussé (80°C à 95°C à 80 Pa) pour fournir 663.0 g d'ester 4-formyl-nonanique de méthyle à 82.7 %. (Rendement : 2.74 mol ; 70 %).

### Exemple 2 : Synthèse du 5-pentyl-tetrahydro-pyran-2-one (9):

Dans un ballon de quatre litres avec agitation mécanique et thermomètre, on charge 1560.0 g d'éthanol et 663.0 g (max. 2.74 mol) de 4-formyl-nonanique ester de méthyle à 82.7 % obtenu dans l'exemple 1 et refroidi à 0°C. Ensuite, on ajoute pendant quatre heures 41.6 g (1.10 mol) de borohydrure de sodium en petites portions sans dépasser 5°C dans la masse. On continue d'agiter après la fin de l'addition pendant encore 24 heures à 0°C. On ajoute goutte à goutte 1900.0 g d'une solution aqueuse d'hydroxyde de sodium à 10 % en deux heures sans dépasser 10°C dans la masse. Un précipité blanc se forme. L'agitation est poursuivie jusqu'à dissolution complète du précipité (environ deux heures). On met le ballon sous un vide d'environ 3990 Pa et on distille l'éthanol. La phase aqueuse obtenue est extraite deux fois avec 200 ml d'un mélange de toluène et d'hexane (50 : 50) pour enlever des produits neutres. La phase aqueuse est ensuite refroidie à 0°C. On acidifie avec 1900.0 g d'acide chlorhydrique à 10 % pendant trois heures sans dépasser 10°C dans la masse. Après la fin de l'addition, l'agitation est poursuivie pendant quatre heures en laissant remonter la température du milieu réactionnel à température ambiante (20°C). On laisse décanter et on récupère la phase organique. La phase aqueuse est extraite deux fois avec 200 ml de toluène. Les phases organiques sont réunies et elles sont lavées deux fois avec 100 ml d'eau et une fois avec 200 ml d'eau salée, puis concentrées sous pression réduite (3990 Pa). On obtient 696.0 g d'un produit brut qui est ensuite blanchi à 133 Pa pour fournir 468.0 g de 5-penthyl-tetrahydro-pyran-2-one à 86.1 %. Après une distillation via une colonne Vigreux, on obtient une fraction de coeur de 289.0 g (1.70 mol) de 5-pentyl-tetrahydro-pyran-2-one à 98.3 % (90-2°C / 27 Pa) avec un rendement de 61 %.

### Exemple 3 : Synthèse du 4-formyl-undécanoïque ester de méthyle (6):

Dans un ballon d'un litre avec agitation magnétique, séparateur d'eau, thermomètre et ampoule d'introduction, on charge 104.4 g (1.20 mol) de morpholine et 210.0 g de cyclohexane. Le mélange est porté à une température de 65°C à 70°C. On ajoute en quatre heures 142.0 g (0.99 mol) de nonanal. On obtient ainsi rapidement la température d'ébullition (80°C-84°C au début de l'addition puis 87°C vers la fin de l'opération.) L'eau formée pendant la réaction est récupérée dans un séparateur d'eau. Le milieu réactionnel est ensuite refroidi et le cyclohexane et l'excès de morpholine sont distillés sous pression réduite (environ 4400 Pa) sans dépasser 60°C dans la masse. On refroidit à 40°C, on porte à pression ambiante et on ajoute 1.5 g d'hydroxyde de potassium dans 20 ml de méthanol à la 4-non-1 (E/Z)-enyl-morpholine. Le mélange est porté à 50°C et 85.5 g (0.99 mol) d'acrylate de méthyle sont ajoutés pendant une heure. On agite quinze heures à 50°C puis on ajoute encore 43.0 g (0.50 mol) d'acrylate de méthyle en une heure et trente minutes. Après la fin de l'addition, on agite encore douze heures à 60°C. La température du milieu réactionnel est augmentée à 80°C. On ajoute goutte à goutte en deux heures à cette température une solution aqueuse d'acide acétique tamponnée à pH 4. (195.7 g acide acétique, 71.7 g acétate de sodium trihydrate et 232.6 g eau). L'agitation est continuée pendant encore quatre heures. On laisse décanter et on sépare les phases. La phase aqueuse est extraite deux fois avec 150 ml de toluène. On réunit les phases organiques et on les lave trois fois avec 100 ml d'eau et une fois avec 100 ml d'eau salée. On sèche sur sulfate de magnésium. Après évaporation du solvant sous pression réduite (3990 Pa) sans dépasser 50°C dans la masse, on obtient 237.0 g d'oxo-ester brut qui est blanchi sous vide poussé (70°C à 120°C à 93 Pa) pour fournir 152.1 g de 4-formyl-undecanoïque ester de méthyle à 91.0 %. (Rendement : 0.61 mol ; 61 %)

### Exemple 4 : Synthèse du 2-Heptyl-pentane-1,5-diol (10):

Dans un ballon d'un litre avec agitation magnétique, thermomètre et ampoule d'introduction, on charge 390.0 g d'isopropanol et 22.4 g (0.59 mol) de borohydrure de sodium. On ajoute goutte à goutte en une heure 148.4 g (0.59 mol) de 4-formyl-undécanoïque ester de méthyle (6) à 91 %. La température dans la masse monte jusqu'à 35°C pendant l'addition. L'agitation est continuée pendant 29 heures en laissant redescendre la température dans la masse à température ambiante (environ 20°C). Après avoir refroidi le milieu réactionnel à 5°C, on ajoute 8.6 g d'acétone sans dépasser 10°C dans la masse. Le milieu réactionnel est refroidi à nouveau à 5°C et 240.0 g d'acide chlorhydrique sont ajoutés en une heure sans dépasser 10°C dans la masse. On ajoute 250.0 g d'eau pour faciliter la séparation des phases. La phase aqueuse est extraite deux fois avec 200 ml de toluène. On réunit les phases organiques et on les lave une fois avec 100.0 ml d'eau et une fois avec 100 ml d'eau salée. On sèche sur sulfate de magnésium et on filtre. Le solvant est évaporé sous pression réduite (3990 Pa) sans dépasser 50°C dans la masse. On obtient 118.0 g (0.42 mol) de 2-Heptyl-pentane-1,5-diol (10) à 72 % qui sont engagés directement dans l'étape de cyclisation.

### Exemple 5 : Synthèse du 3-Heptyl-tetrahydro-pyranne (11)

Dans un ballon de 500 ml avec agitation magnétique, réfrigérant, thermomètre et ampoule d'introduction, on charge 60.6 (0.21 mol) de 2-Heptyl-pentane-1,5-diol et ajoute rapidement 52.6 g d'acide phosphorique à 85 %. La température dans le milieu réactionnel monte jusqu'à 60°C pendant l'addition. Le mélange réactionnel est porté au reflux (127°C) et il est agité pendant quatre heures. On refroidit à température ambiante (environ 20°C) et on ajoute 150 ml d'eau. L'agitation est augmentée et elle est continuée pendant 30 minutes. On sépare les phases et on extrait la phase aqueuse une fois avec 100 ml de méthyl t-butyl éther. Les phases organiques sont réunies et elles sont lavées deux fois avec 50 ml d'une solution saturée de bicarbonate de sodium et une fois avec 50 ml d'eau salée. On sèche, on filtre et on évapore le solvant sous pression réduite (3990 Pa) sans dépasser 50°C dans la masse. Le produit brut est distillé ensuite via une colonne Vigreux. La fraction de coeur contient 28.1 g (0.18 mol) de 3-Heptyl-tetrahydro-pyranne à 96 %. (Température d'ébullition : 100°C à 930 Pa). Le rendement est à 73 %.

Les analyses des spectres infra rouges, RMN et de masse des composés obtenus montrent qu'ils correspondent aux structures des composés attendus.

### Exemple 6 : Evaluation olfactive

Dans un premier temps, les caractéristiques odorantes de plusieurs composés purs ont été évaluées par un panel. Le panel d'évaluation est composé de plusieurs professionnels, évaluant qualitativement chaque composé. Les composés ont été décrits comme fruités, lactoniques avec des notes de feuilles de figuier ou de coriandre. Les résultats des évaluations sont rassemblés ci-après :
- 5-propyl-tétrahydro-2H-pyran-2-one : lactonique, noix de coco, feuille de figuier, dans le registre de l'octahydrocoumarine
- 5-pentyl-tétrahydro-2H-pyran-2-one : lactonique, citronné, vert, verveine
- 3-heptyl-tétrahydropyranne : lactonique, vert, aldéhyde, feuille de coriandre, mandarine, banane
- 3-benzyl-tétrahydropyranne : géranium, boisé, phénolique, lactonique.

La 5-pentyl-tetrahydro-2H-pyran-2-one et la 5-heptyl-tetrahydro-2H-pyran-2-one ont ensuite été testées en base shampooing nacré (compositions 1, et 2 & 3, respectivement). Leur impact odorant a été jugé en comparant des compositions parfumantes sans et avec chaque composé. Le 3-benzyl-tetrahydropyranne a été testé en base shampooing nacré et en base assouplissant concentrée (compositions 4 et 5). Son impact odorant a été comparé avec celui de l'acétate de styrallyle.

### Composition N° 1

### Tiaré

### (application 0.5 % en poids/poids d'une base shampooing nacré)

| Composant | *Essai 1 (g)* | *Essai 2 (g)* |
|---|---|---|
| Acétate de benzyle | 60.0 | 60.0 |
| Alcool cinnamique Synth. Prime ⁽¹⁾ | 6.0 | 6.0 |
| Alcool phenyléthylique | 80.0 | 80.0 |
| Antranylate de méthyle | 20.0 | 20.0 |
| Benzoate de méthyle | 2.0 | 2.0 |
| Citronellol à 98 % | 60.0 | 60.0 |
| Coumarine | 4.0 | 4.0 |
| Géraniol pur | 56.0 | 56.0 |
| Dihydro jasmonate de méthyle | 200.0 | 200.0 |
| Héliotropine cristallisée | 8.0 | 8.0 |
| Isoeugénol | 3.0 | 3.0 |
| Linalol | 100.0 | 100.0 |
| Lyral ⁽²⁾ | 130.0 | 130.0 |
| Méthylionanthème | 40.0 | 40.0 |
| Salicylate de Benzyle | 200.0 | 200.0 |
| Folione ⁽³⁾ | 5.0 | 5.0 |
| Indole à 10 % dans DPG | 10.0 | 10.0 |
| Méthyl paracrésol à 10 % DPG | 6.0 | 6.0 |
| Dipropylèneglycol | 10.0 | 0.0 |
| 5-pentyl-tetrahydro-2H-pyran-2-one | 0.0 | 10.0 |
| Total | 1000.0 | 1000.0 |

| | | |
|---|---|---|
| ⁽¹⁾ Origine : Givaudan, Suisse. ⁽²⁾ 4-(4-Hydroxy-4-methyl pentyl)-3-cyclohexene-1-carbaldehyde ; origine : International Flavors & Fragrances Inc., USA. ⁽³⁾ Methyl 2-octynoate, origine : Givaudan, Suisse. | | |

### Composition N° 2

### Muguet

### (application 0.5 % en poids d'une base shampooing nacré)

| Composant | *Essai 1 (g)* | *Essai 2 (g)* |
|---|---|---|
| Acétate de benzyle | 56.0 | 56.0 |
| Alcool phényléthylique | 445.0 | 445.0 |
| Aldéhyde alpha hexylcinnamique | 100.0 | 100.0 |
| Citronellol à 98 % | 80.0 | 80.0 |
| Dihydro jasmonate de méthyle | 100.0 | 100.0 |
| Lilial ⁽¹⁾ | 85.0 | 85.0 |
| Linalol | 110.0 | 110.0 |
| Triplal ⁽²⁾ | 1.0 | 1.0 |
| cis-3-hexénol à 10 % dans DPG | 6.0 | 6.0 |
| Indole à 10 % dans DPG | 6.0 | 6.0 |
| Vanilline à 10 % dans DPG | 6.0 | 6.0 |
| Dipropylèneglycol | 10.0 | 0.0 |
| 5-Heptyl-tetrahydro-2H-pyran-2-one | 0.0 | 10.0 |
| Total | 1000.0 | 1000.0 |

| | | |
|---|---|---|
| ⁽¹⁾ 2-Methyl-3(4-tert-butylphenyl)propanal, origine : Givaudan, Suisse. ⁽²⁾ 2,4-Dimethyl-3-cyclohexene-1-carbaldehyde, origine : International Flavors & Fragrances Inc., USA. | | |

### Composition N° 3

### Pêche

### (application 0.5 % en poids d'une base shampooing nacré)

| Composant | *Essai 1 (g)* | *Essai 2 (g)* |
|---|---|---|
| Acétate d'isoamyle | 10.0 | 10.0 |
| Acétate de benzyle | 65.0 | 65.0 |
| Alcool phényléthylique | 40.0 | 40.0 |
| Caproate d'allyle | 5.0 | 5.0 |
| Givescone ⁽¹⁾ | 5.0 | 5.0 |
| Dihydro jasmonate de méthyle | 145.0 | 145.0 |
| *Cis*-3-hexénol | 4.0 | 4.0 |
| Alpha-ionone | 15.0 | 15.0 |
| *cis*-Jasmone ⁽²⁾ | 2.0 | 2.0 |
| Linalol | 175.0 | 175.0 |
| Neofolione ⁽³⁾ | 1.0 | 1.0 |
| Frambinone | 3.0 | 3.0 |
| γ-Nonalactone | 10.0 | 10.0 |
| Tetrahydrolinalol | 260.0 | 260.0 |
| Salicylate d'hexyle | 155.0 | 155.0 |
| Corps M020 ⁽⁴⁾ | 80.0 | 80.0 |
| Butyrate d'éthyle à 10 % dans DPG | 5.0 | 5.0 |
| Dipropylèneglycol | 20.0 | 0.0 |
| 5-Heptyl-tetrahydro-2H-pyran-2-one | 0.0 | 20.0 |
| Total | 1000.0 | 1000.0 |

| | | |
|---|---|---|
| ⁽¹⁾ Ethyl 2-ethyl-6,6-dimethyl-2-cyclohexène-carboxylate and Ethyl 2,3,6,6-teramethyl-2-cyclohexene-carboxylate, origine : Givaudan, Suisse. ⁽²⁾ Origine : Givaudan, Suisse. ⁽³⁾ Methyl 2-nonenoate, origine : Givaudan, Suisse. ⁽⁴⁾ Spécialité de V. Mane Fils | | |

### Composition N° 4

### Mûre Myrtille

### (application 0.5 % en poids d'une base assouplissant)

| Composant | *Essai 1 (g)* | *Essai 2 (g)* |
|---|---|---|
| Cassis MGO ⁽¹⁾ | 30.0 | 30.0 |
| Acétate d'isoamyle | 6.0 | 6.0 |
| Acétate de Benzyle | 20.0 | 20.0 |
| Ethyle acétyle acétate | 25.0 | 25.0 |
| Aldéhyde alpha hexylecinnamique | 55.0 | 55.0 |
| Aldéhyde laurique | 2.0 | 2.0 |
| Gamma-undécalactone | 30.0 | 30.0 |
| ² | 45.0 | 45.0 |
| Bacdanol (2) | 10.0 | 10.0 |
| Benjoin de Siam à 50 % dans EMK | 2.0 | 2.0 |
| Butyrate de butyle | 3.0 | 3.0 |
| Cinnamate d'éthyle | 4.0 | 4.0 |
| Citron Ess. Italie | 60.0 | 60.0 |
| Dipropylène glycol | 186.0 | 186.0 |
| cis-3-Hexénol | 5.0 | 5.0 |
| α-Ionone | 12.0 | 12.0 |
| β-Ionone | 18.0 | 18.0 |
| Lilial ⁽³⁾ | 30.0 | 30.0 |
| Limette Ess. distillée Haïti | 20.0 | 20.0 |
| Linalol | 20.0 | 20.0 |
| Méthylionanthème Super ⁽⁴⁾ | 35.0 | 35.0 |
| Musc T - éthylène brassylate⁽⁵⁾ | 200.0 | 200.0 |
| Orange Ess. Brésil | 30.0 | 30.0 |
| Frambinone ⁽⁶⁾ | 10.0 | 10.0 |
| Salicylate de benzyle | 25.0 | 25.0 |
| Undécavertol ⁽⁷⁾ | 4.0 | 4.0 |
| Ethyl maltol - Veltol Plus ⁽⁸⁾ | 2.0 | 2.0 |
| Verdox ⁽⁹⁾ | 35.0 | 35.0 |
| Aldéhyde benzoïque sans toluène à 10 % dans DPG | 5.0 | 5.0 |
| Cumin Ess. | 2.0 | 2.0 |
| Damascenones à 10 % dans DPG ⁽¹⁰⁾ | 7.0 | 7.0 |
| Folione à 10 % dans DPG ⁽¹¹⁾ | 4.0 | 4.0 |
| Galbanum Ess. | 4.0 | 4.0 |
| Girofle clou Ess. | 4.0 | 4.0 |
| Melonal à 10 % dans DPG ⁽¹²⁾ | 2.0 | 2.0 |
| 6-cis-nonenol à 1 % dans DPG | 5.0 | 5.0 |
| Oxane à 10 % dans DPG ⁽¹³⁾ | 15.0 | 15.0 |
| Diméthyl sulfure à 1 % dans DPG | 15.0 | 15.0 |
| Acétate de styrallyle | 25.0 | 0.0 |
| 3-benzyl-tetrahydro-pyranne | 0.0 | 25.0 |
| Total | 1000.0 | 1000.0 |

| | | |
|---|---|---|
| ⁽¹⁾ Spécialité de V. Mane Fils ⁽²⁾ 2-Ethyl-4-(2,3,3-trimethyl-3-cyclopentyl-1-yl)-2-buten-1-ol, origine : International Flavors & Fragrances Inc. USA. ⁽³⁾ 2-Methyl-3(4-tert-butylphenyl)propanal, origine : Givaudan, Suisse. ⁽⁴⁾ Origine : Givaudan, Suisse. ⁽⁵⁾ 1,4-Dioxacycloheptadecane-5,17-dione ⁽⁶⁾ 4-(4-Hydroxyphenyl)-2-butanone ⁽⁷⁾ 4-methyl-3-decen-5-ol, origine : Givaudan, Suisse. ⁽⁸⁾ 2-Ethyl-3-hydroxy-pyran-4-one ⁽⁹⁾ 2-tert-butyl-cyclohexan-1-yl acetate, origine : International Flavors & Fragrances Inc., USA. ⁽¹⁰⁾ 1-(2,6,6-Trimethyl-1,3-cyclohexadien-1-yl)-2-buten-1-one (isomères), origine : Firmenich, Suisse. ⁽¹¹⁾ Methyl 2-octynoate, origine : Givaudan, Suisse) ⁽¹²⁾ 2,6-Dimethyl-5hepten-1-al, origine : Givaudan, Suisse. ⁽¹³⁾ cis-2-Methyl-4-propyl-1,3-oxathiane, origine : Firmenich, Suisse. | | |

### Composition N° 5

### Goyave

### (application 0.5 % en poids d'une base shampooing nacré et en base assouplissant)

| Composant | *Essai 1 (g)* | *Essai 2 (g)* |
|---|---|---|
| Cassis MGO ⁽¹⁾ | 6.0 | 6.0 |
| Acétate de cis-3-Hexényle VMF ⁽²⁾ | 70.0 | 70.0 |
| Diméthylbenzylcarbinyle acétate | 12.0 | 12.0 |
| Acétate d'éthyle | 6.0 | 6.0 |
| Acétate d'hexyle | 6.0 | 6.0 |
| Acétate de phényléthyle | 10.0 | 10.0 |
| Ethyle acétyle acétate | 170.0 | 170.0 |
| Alcool Phényléthylique | 50.0 | 50.0 |
| γ-Undécalactone | 40.0 | 40.0 |
| cis-Isoambrettolide | 20.0 | 20.0 |
| Butyrate d'éthyle | 24.0 | 24.0 |
| Cinnamate d'éthyle | 140.0 | 140.0 |
| Dipropylène glycol | 124.0 | 124.0 |
| Eugénol Rect. VMF ⁽²⁾ | 30.0 | 30.0 |
| γ-Décalactone | 30.0 | 30.0 |
| cis-3-Hexénol | 14.0 | 14.0 |
| Isobutyrate de cis-3-hexényle VMF ⁽²⁾ | 30.0 | 30.0 |
| β-Ionone | 12.0 | 12.0 |
| Musc T éthylène brassylate ⁽³⁾ | 30.0 | 30.0 |
| Terpènes Orange Brésil | 20.0 | 20.0 |
| γ-Nonalactone | 10.0 | 10.0 |
| Tetrahydrolinalol | 50.0 | 50.0 |
| Vanilline | 14.0 | 14.0 |
| Pamplemousse à 10 % dans DPG ⁽⁴⁾ | 6.0 | 6.0 |
| Méthyle anthranilate à 10 % dans DPG | 8.0 | 8.0 |
| Ethylmaltol à 1 % dans DPG ⁽⁵⁾ | 20.0 | 20.0 |
| Corps M290 à 10 % dans DPG ⁽⁶⁾ | 8.0 | 8.0 |
| Acétate de Styrallyle | 8.0 | 0.0 |
| 3-Benzyle-tetrahydro-pyranne | 0.0 | 8.0 |
| Total | 1000.0 | 1000.0 |

| | | |
|---|---|---|
| ⁽¹⁾ Spécialité de V. Mane Fils ⁽²⁾ Origine : V. Mane Fils ⁽³⁾ 1,4-Dioxacycloheptadecane-5,17-dione ⁽⁴⁾ Spécialité de V. Mane Fils ⁽⁵⁾ 2-Ethyl-3-hydroxy-pyran-4-one ⁽⁶⁾ Spécialité de V. Mane Fils | | |

Dans chaque cas, les évaluations de l'impact olfactif ont été effectuées à tₒ, t₊₆ₕ, t₊₂₄ₕ pour évaluer les notes de tête, de coeur et de fond.

La 5-pentyl-tetrahydro-2H-pyran-2-one apporte à la composition N°1 un aspect plus aéré et clair.

La 5-heptyl-tetrahydro-2H-pyran-2-one apporte à la composition N°2 un coté plus transparent et vert. Par contre, dans la composition N°3, elle apporte un aspect moins vert, mais un coté plus chair de fruit.

Le 3-benzyl-tetrahydro-pyranne apporte à la composition N°4 une note rhubarbe plus naturelle avec un coté vert astringent boisé.

Il apporte à la composition N°5 plus de volume et puissance en améliorant la note de goyave.

La perte d'intensité avec le temps semble assez linéaire, sans laisser apparaître un important changement de caractéristique odorante.

Les résultats de ces évaluations montrent sans le moindre doute que les composés décrits ci-dessus présentent des caractéristiques olfactives intéressantes, qui trouveront une application en particulier dans les cosmétiques, la parfumerie, les produits d'entretien, et d'une manière générale dans toute composition odorante ou dont il est souhaitée de masquer ou neutraliser l'odeur.

## Revendications

1. Utilisation d'un composé tétrahydropyra(no)ne substitué en position bêta par rapport à l'oxygène du cycle en tant qu'agent odorant, ledit composé étant représenté par la formule suivante : 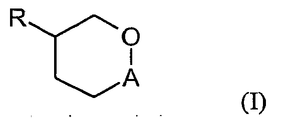 dans laquelle le substituant R représente un radical alkyle linéaire CH₃-(CH₂)ₙ- avec n = 2 à 10 inclus, (CH₃)₂CH- ou C₆H₅-(CH₂)ₘ-, avec m = 0 ou 1;
ou 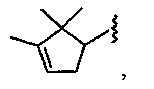 et dans laquelle A représente -CH₂- ou -CO-.

2. Utilisation selon la revendication 1, **caractérisée en ce que** ledit composé est un composé de formule (Ia) correspondant à la formule (I) dans laquelle A représente -CO-.

3. Utilisation selon la revendication 1, **caractérisée en ce que** ledit composé est un composé de formule (Ib) correspondant à la formule (I) dans laquelle A représente -CH₂-.

4. Utilisation selon la revendication 1, **caractérisée en ce que** ledit composé est choisi parmi :
5-pentyl-tétrahydro-pyran-2-one,
3-heptyl-tétrahydro-pyranne,
5-heptyl-tétrahydro-pyran-2-one, et
3-benzyl-tetrahydro-pyranne.

5. Composé nouveau de formule (I) 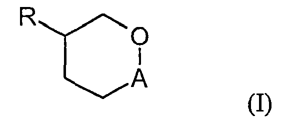 dans laquelle
le substituant R représente un radical alkyle linéaire CH₃-(CH₂)ₙ- avec n = 6 à 10 inclus,
ou 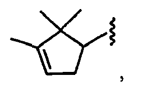 lorsque A représente -CH₂- ,
et
le substituant R représente un radical alkyle linéaire CH₃-(CH₂)ₙ- avec n = 8, ou C₆H₅-
ou 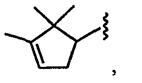 lorsque A représente -CO-.

6. Procédé de synthèse d'un composé de formule (I) 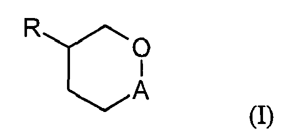 dans laquelle le substituant R représente un radical alkyle linéaire CH₃-(CH₂)ₙ- avec n = 2 à 10 inclus, (CH₃)₂CH- ou C₆H₅-(CH₂)ₘ-, avec m = 0 ou 1;
ou 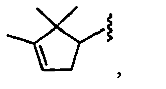 et dans laquelle A représente -CH₂- ou -CO-,
le procédé comprenant la réduction d'un oxo-ester de formule (6) 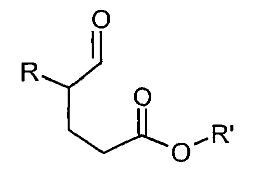 dans laquelle R a la même signification que dans la formule (I) et R' représente CH₃ ou C₂H₅- ou ester d'alcool supérieur.

7. Procédé selon la revendication 6, **caractérisé en ce que** la réduction de l'oxo-ester a lieu avec un agent réducteur, par exemple le borohydrure de sodium, à basse température, pour former un hydroxy-ester, suivie d'une saponification avec une base appropriée, puis une hydrolyse avec un acide approprié, suivie d'une cyclisation pour former un composé de formule (I) dans laquelle A représente -CO-.

8. Procédé selon la revendication 6, **caractérisé en ce que** la réduction de l'oxo-ester a lieu avec un agent réducteur, par exemple le borohydrure de sodium, à haute température, pour former un diol, suivie d'une cyclisation pour former un composé de formule (I) dans laquelle A représente -CH₂-.

9. Procédé selon l'une quelconque des revendications 6 à 8, **caractérisé en ce que** la réduction de l'oxo-ester est précédée de la condensation d'une amine secondaire avec un aldéhyde, pour former une énamine, suivie de la condensation de l'énamine obtenue avec un acrylate, pour former un énamine ester qui est hydrolysé en oxo-ester.

10. Composition comprenant un produit de base et une quantité efficace d'un composé tétrahydropyra(no)ne substitué en position bêta par rapport à l'oxygène du cycle en tant qu'agent odorant, tel que défini dans l'une des revendications 1 à 4.

11. Composition selon la revendication 10, dans laquelle ledit agent odorant est utilisé pour masquer ou neutraliser des odeurs.

12. Composition selon la revendication 10 **caractérisée en ce qu'**elle est choisie parmi les compositions de parfumerie au sens habituel du terme, telles que bases et concentrés parfumants, eaux de Cologne, eaux de toilette, parfums et produits similaires; les compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, antitranspirants et désodorisants corporels, lotions et crèmes de rasage, savons, crèmes, dentifrices, bains de bouche, pommades, et produits similaires; et les produits d'entretien, tels qu'assouplissants, détergents, lessives, désodorisants d'ambiance, et produits similaires.

## Claims

1. Use of a tetrahydropyran(one) compound substituted in the beta position relative to the ring oxygen, as a fragrant agent, said compound being represented by the following formula:
in which the substituent R represents a linear alkyl radical CH₃-(CH₂)ₙ- with n = 2 to 10 inclusive, (CH₃)₂CH-
or C₆H₅-(CH₂)ₘ-, with m = O or 1;
or 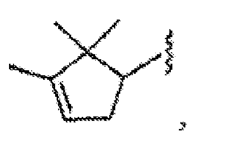
and in which A represents -CH₂- or -CO-.

2. Use according to claim 1, **characterized in that** said compound is a compound of formula (1a) corresponding to formula (I) in which A represents -CO-.

3. Use according to claim 1, **characterized in that**said compound is a compound of formula (Ib) corresponding to formula (I) in which A represents -CH₂-.

4. Use according to claim 1, **characterized in that** said compound is chosen from:
5-pentyltetrahydropyran-2 -one,
3-heptyltetrahydropyran,
5-heptyltetrahydropyran-2-one,
3-benzyltetrahydropyran.

5. Compound of formula (I) 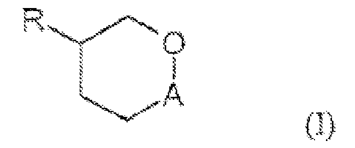 in which the substituent R represents a linear alkyl radical CH₃-(CH₂)ₙ- with n = 6 to 10 inclusive,
or 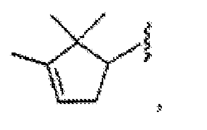 when A represents -CH₂-,
and the substituent R represents a linear alkyl radical CH₃-(CH₂)ₙ- with n = 8, or C₆H₅
or 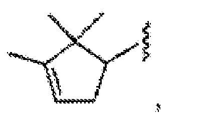 when A represents -CO-.

6. Process for synthesizing a compound of formula (I) 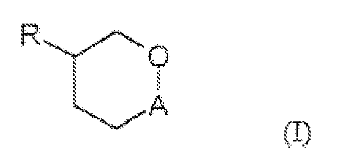 in which the substituent R represents a linear alkyl radical CH₃-(CH₂)ₙ with n = 2 to 10 inclusive, (CH₃)₂CH-
or C₆H₅-(CH₂)ₘ-, with m = O or 1;
or 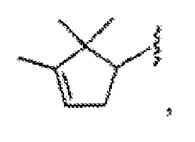 and in which A represents -CH₂- or -CO-, the process comprising the reduction of an oxo ester of formula (6) 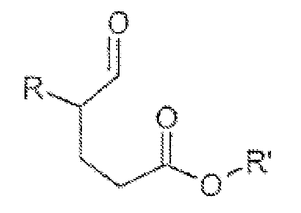 in which R has the same meaning as in formula (I) and R' represents CH₃ or C₂H₅- or a higher alcohol ester.

7. Process according to claim 6, **characterized in that** the reduction of the oxo ester takes place with a reducing agent, for example sodium borohydride, at low temperature, to form a hydroxy ester, followed by saponification with a suitable base, and then hydrolysis with a suitable acid, followed by cyclization to form a compound of formula (I) in which A represents -CO-.

8. Process according to claim 6, **characterized in that** the reduction of the oxo ester takes place with a reducing agent, for example sodium borohydride, at high temperature, to form a diol, followed by cyclization to form a compound of formula (I) in which A represents -CH₂-.

9. Process according to any one of claims 6 to 8, **characterized in that** the reduction of the oxo ester is preceded by the condensation of a secondary amine with an aldehyde, to form an enamine, followed by condensation of the enamine obtained with an acrylate, to form an enamine ester which is hydrolyzed to an oxo ester.

10. Composition comprising a base product and an effective amount of a tetrahydropyran(one) compound substituted in the beta position relative to the ring oxygen, as a fragrant agent, as defined in one of claims 1 to 4.

11. Composition according to claim 10, in which said fragrant agent is used to mask or neutralize odors.

12. Composition according to claim 10, **characterized in that** it is chosen from perfumery compositions in the usual sense of the term, such as fragrancing bases and concentrates, eaux de Cologne, eaux de toilette, fragrances and similar products; topical compositions - in particular cosmetic compositions - such as facial and body creams, talc powders, hair oils, shampoos, hair lotions, bath salts and oils, shower and bath gels, toiletry soaps, antiperspirants and body deodorants, shaving lotions and creams, soaps, creams, toothpastes, mouthwashes, salves, and similar products; and maintenance products, such as softeners, detergents, laundry washing products, ambient deodorizers, and similar products.

## Patentansprüche

1. Verwendung einer Tetrahydropyra(no)nverbindung, die in beta-Stellung zum Ringsauerstoff substituiert ist, als Duftstoff, wobei die Verbindung durch die folgende Formel wiedergegeben wird: 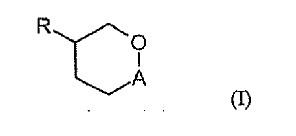 worin der Substituent R für einen linearen Alkylrest CH₃-(CH₂)n mit n = 2 bis 10 inklusive, (CH₃)₂CH- oder C₆H₅-(CH₂)m- mit m = 0 oder 1 oder 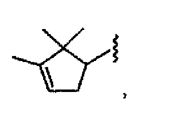 steht
und A für -CH₂- oder -CO- steht.

2. Verwendung nach Anspruch 1, **dadurch gekennzeichnet, dass** es sich bei der Verbindung um eine Formel (Ia) handelt, die der Formel (I), worin A für -CO- steht, entspricht.

3. Verwendung nach Anspruch 1, **dadurch gekennzeichnet, dass** es sich bei der Verbindung um eine Formel (Ib) handelt, die der Formel (I), worin A für -CH₂- steht, entspricht.

4. Verwendung nach Anspruch 1, **dadurch gekennzeichnet, dass** die Verbindung unter
5-Pentyltetrahydropyran-2-on,
3-Heptyltetrahydropyran,
5-Heptyltetrahydropyran-2-on und
3-Benzyltetrahydropyran
ausgewählt ist.

5. Neue Verbindung der Formel (I) 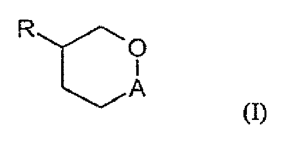 worin der Substituent R für einen linearen Alkylrest CH₃-(CH₂)ₙ mit n = 6 bis 10 inklusive oder 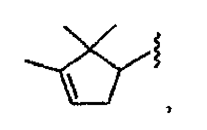 steht, wenn A für -CH₂- steht,
und
der Substituent R für einen linearen Alkylrest CH₃-(CH₂)n mit n = 8 oder C₆H₅- oder 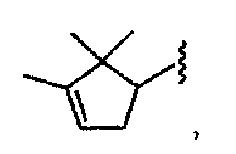 steht, wenn A für -CO- steht.

6. Verfahren zur Synthese einer Verbindung der Formel (I) 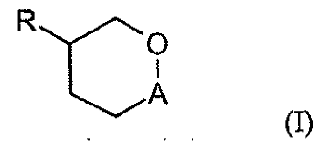 worin der Substituent R für einen linearen Alkylrest CH₃-(CH₂)ₙ mit n = 2 bis 10 inklusive, (CH₃)₂CH- oder C₆H₅-(CH₂)m- mit m = 0 oder 1 oder 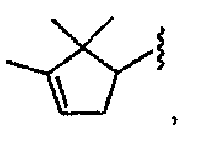 steht
und A für -CH₂- oder -CO- steht,
bei dem man einen Oxoester der Formel (6) 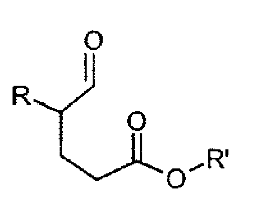 worin R die gleiche Bedeutung wie in Formel (I) besitzt und R' für CH₃ oder C₂H₅- oder eine Ester eines höheren Alkohols steht, reduziert.

7. Verfahren nach Anspruch 6, **dadurch gekennzeichnet, dass** die Reduktion des Oxoesters mit einem Reduktionsmittel, beispielsweise Natriumborhydrid, bei niedriger Temperatur zur Bildung eines Hydroxyesters gefolgt von einer Verseifung mit einer geeigneten Base und dann einer Hydrolyse mit einer geeigneten Säure gefolgt von einer Cyclisierung zur Bildung einer Verbindung der Formel (I), in der A für -CO- steht, erfolgt.

8. Verfahren nach Anspruch 6, **dadurch gekennzeichnet, dass** die Reduktion des Oxoesters mit einem Reduktionsmittel, beispielsweise Natriumborhydrid, bei hoher Temperatur zur Bildung eines Diols gefolgt von einer Cyclisierung zur Bildung einer Verbindung der Formel (I), in der A für -CH₂- steht, erfolgt.

9. Verfahren nach einem der Ansprüche 6 bis 8, **dadurch gekennzeichnet, dass** man vor der Reduktion des Oxoesters ein sekundäres Amin mit einem Aldehyd zu einem Enamin kondensiert und das erhaltene Enamin mit einem Acrylat zu einem Enaminester kondensiert, welchen man zum Oxoester hydrolysiert.

10. Zusammensetzung, enthaltend ein Basisprodukt und eine wirksame Menge einer Tetrahydropyra(no)nverbindung, die in beta-Stellung zum Ringsauerstoff substituiert ist, als Duftstoff gemäß einem der Ansprüche 1 bis 4.

11. Zusammensetzung nach Anspruch 10, worin der Duftstoff zur Maskierung oder Neutralisierung von Gerüchen verwendet wird.

12. Zusammensetzung nach Anspruch 10, **dadurch gekennzeichnet, dass** sie unter Parfümeriezusammensetzungen im üblichen Sinne des Begriffs, wie Parfümbasen und -konzentrate, Eaux de Cologne, Eaux de Toilette, Parfüms und ähnlichen Produkten; topischen Zusammensetzungen - insbesondere Kosmetika - wie Gesichts- und Körpercremes, Talkumpudern, Haarölen, Shampoos, Haarlotionen, Badesalzen und -ölen, Dusch- und Badegelen, Toilettenseifen, Körperantitranspirantien und -deodorantien, Rasierlotionen und - cremes, Seifen, Cremes, Zahnpasten, Mundwässern, Pomaden und ähnlichen Produkten und Pflegeprodukten wie Weichmacher, Reinigungsmittel, Waschmittel, Raumdeodorant und ähnlichen Produkten ausgewählt ist.
